# EUROPEAN PATENT SPECIFICATION

(11) **EP 4 193 004 B1**
(45) Date of publication and mention of the grant of the patent: **27.03.2024**
(21) Application number: 21742402.7
(22) Date of filing: 13.07.2021
(51) Int. Cl.: C25B 3/23, C07C 51/145, C07C 59/01, C25B 3/07

(54) **METHOD FOR PREPARING 3-HYDROXY-3-METHYLBUTYRIC ACID (HMB) OR SALTS THEREOF**
VERFAHREN ZUR HERSTELLUNG VON 3-HYDROXY-3-METHYLBUTTERSÄURE (HMB) ODER SALZEN DAVON
PROCÉDÉ DE PRÉPARATION D'ACIDE 3-HYDROXY-3-MÉTHYLBUTYRIQUE (HMB) OU DE SES SELS

(30) Priority: 05.08.2020 EP 20189649
(43) Date of publication of application: 14.06.2023
(73) Proprietor: Evonik Operations GmbH, 45128 Essen (DE)
(72) Inventor: STAHL, Timo, 63694 Limeshain (DE); STENNER, Patrik, 63452 Hanau (DE); RONNEBURG, Axel, 63457 Hanau (DE); TEICHMANN, Julian, 60438 Frankfurt am Main (DE)
(74) Representative: Evonik Patent Association
(86) International application number: PCT/EP2021/069423
(87) International publication number: WO 2022/028821

(56) References cited:
- D. D. COFFMAN ET AL: "Syntheses by Free-radical Reactions. V. A New Synthesis of Carboxylic Acids", JOURNAL OF THE AMERICAN CHEMICAL SOCIETY, vol. 80, no. 11, 1 June 1958 (1958-06-01), pages 2882-2887, XP055764115, US ISSN: 0002-7863, DOI: 10.1021/ja01544a072

## Description

### Field of the Invention

The present invention pertains to a new process for preparing 3-hydroxy-3-methylbutyric acid (HMB) and salts of HMB.

### Background of the Invention

3-Hydroxy-3-methylbutyric acid (HMB) is known as a metabolite of the essential amino acid L-leucine. Due to several positive effects, mainly related to either strengthen, or recovering muscle tissue, HMB is used as a nutritional supplement for humans, in particular in strength- and endurance sports (see: J. Int. Soc. Sports Nutr. 2013, 10, 6). In addition, HMB is used in pharmaceutical applications, such as in the treatment of muscular dystrophy.

The positive effects of HMB, however, are not limited to applications in humans. The use of HMB as feed additive or feed supplement has been described for a number of economically relevant livestock, such as poultry and pigs, see e.g. US 5,087,472 or US 2010/0179112 A1.

The dosage form mainly applied for HMB is the calcium salt, which may be obtained from the free acid. Compared to the free acid, the calcium salt comes with some advantages, such as stability, no odor, and non-corrosive properties.

In the established manufacturing process, the key synthetic reaction step is a Baeyer-Villiger oxidation of diacetone alcohol, whereby the HMB, or its salts, obtained. Sodium hypochloride (NaOCI, described in EP 2 744 489 A1) is used as a typical oxidant in this reaction, thereby yielding the sodium salt of HMB. Neutralization with hydrochloric acid yields HMB as a free acid.

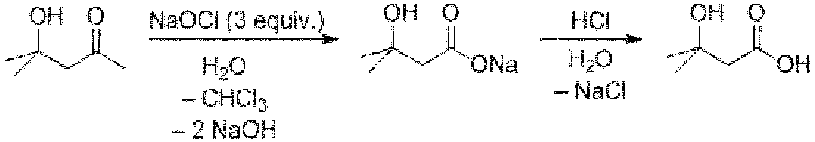

Other reported oxidants include sodium hypobromide (described in CN1417190) or freshly prepared peracetic acid (described in CN103694107). All these reactions come with signficant limitations: a production process based on NaOCI or NaOBr not only produces stoichiometric quantities of salt but also large quantities of undesirable hazardous substances such as chloroform or bromoform. In addition, the yield of the oxidation reaction is rather low. A process based on peracetic acid requires a flammable organic solvent and is as well described by a rather low yield based on both diacetone alcohol and peracetic acid precursors hydrogen peroxide and acetic acid.

In an alternative process, HMB can be synthesized directly from *tert*-butanol and carbon monoxide using "Fenton chemistry" (see D. D. Coffmann, R. Cramer, W. e. Mochel, J. Am. Chem. Soc. 1958 80, 2882-2887). The term "Fenton chemistry" means to form hydroxyl (OH) radicals from hydrogen peroxide with the use of stoichiometric quantities of iron (II) sulphate. In the presence of *tert-*butanol and carbon monoxide, these hydroxyl radicals can undergo a radical reaction to give the desired product.

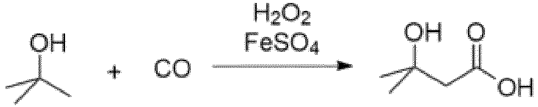

It is precisely the necessary use of iron (II) sulphate that makes this approach uneconomical for two reasons: a) iron (II) sulphate is a comparatively expensive raw material and b) salts are formed stoichiometrically in the course of the reaction that must be disposed of.

Accordingly, it was the objective of the present invention to provide a simple and inexpensive manufacturing process for HMB in which less hazardous byproducts are formed and in which the formation of byproducts is minimized, respectively.

### Summary of the Invention

The inventors have found a new synthesis route to HMB that meets the requirements of the above objective. In particular, the inventors have unexpectedly found that 3-hydroxy-3-methylbutyric acid (HMB), and salts thereof, can be obtained from *tert*-butanol and carbon monoxide in an aqueous reaction mixture simply be applying electrolytic conditions. In other words, compared to the above-mentioned "Fenton"-route, both the function of the iron (II) sulphate and the function of hydrogen peroxide can be completely replaced by electrochemistry in aqueous solution.

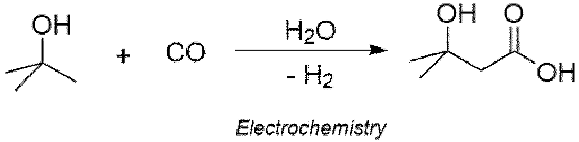

Accordingly, the present invention pertains to a method for preparing 3-hydroxy-3-methylbutyric acid (HMB) or salts thereof from *tert*-butanol and carbon monoxide, wherein *tert*-butanol is reacted in an aqueous mixture with carbon monoxide under electrolytic conditions.

### Detailed Description of the Invention

The term "electrolytic conditions" generally refers to applying an electric potential and current to the respective reaction mixture.

For the method according to the present invention, the electric potential to be applied to the aqueous reaction mixture may be between 1.5 V and 25 V, in particular between 15 V and 23 V and preferably between 18 V and 22 V.

The electrodes may be operated at a current density starting from 1 A/m² up to 10.000 A/m², in particular between 50 A/m² and 5.000 A/m² and preferably between 100 A/m² and 3500 A/m².

A wide variety of electrode materials are known in the art. For the method according to the present invention, electrodes allowing the formation of hydroxyl radicals are particularly suitable. For example, the electric potential may be applied to the aqueous reaction mixture using a diamond anode and/or a steel cathode. In contrast to the established platinum anodes, diamond anodes have a larger potential window and suppress oxygen (gaseous O₂) formation to the advantage of the formation of hydroxyl radicals in aqueous systems.

Form improving the conductivity of the aqueous solution, at least conducting salt may be used in small quantities. Said conducting electrolyte are not consumed during the reaction, but significantly improves the efficiency of hydroxyl formation. The initial amount of the at least one conducting electrolyte present in the aqueous reaction mixture (i.e. the amount of of the at least one conducting electrolyte present in the aqueous reaction mixture at the beginning of the reaction) may between 1 wt.% and 20 wt.%, in particular between 5 wt.% and 15 wt.% and preferably between 10 wt.% and 14 wt%.; based on the amount of *tert*-butanol present in the aqueous reaction mixture.

The HMB reaction product obtainable via the method according to the present invention itself may serve as a conducting electrolyte. In this case, an initial amount of HMB or HMB salt has to be introduced to the reaction mixture before stating the electrochemical reaction.

In general, the at least one conducting electrolyte may be selected from the group consisting of tetraalkyl ammonium hexaflurophosphates, tetraalkyl ammonium tetrafluoroborates, and tetraalkyl imidazolium triflates, and 3-hydroxy-3-methylbutyric acid or salts thereof (HMB), or any combination of these conducting electrolytes. Conducting electrolytes selected from the group consisting of tetrabutyl ammoniumhexafluorophosphate, tetrabutyl ammoniumtetrafluoroborate, 1-butyl-3-methylimidazolium triflate, and 3-hydroxy-3-methylbutyric acid or salts thereof (HMB), or any combination of these conducting electrolytes are particularly suitable.

Preferably, the conducting electrolyte is 1-butyl-3-methylimidazolium triflate.

The carbon monoxide may be provided to the aqueous reaction mixture in gaseous form, e.g. by simply bubbling carbon monoxide through the aqueous reaction mixture. Alternatively, the carbon monoxide may be obtained in *situ* from gaseous carbon dioxide. In the latter case, the carbon dioxide is bubbled through the aqueous reaction mixture and is reduced *in situ,* i.e. under the electrolytic conditions and via electron transfer, to carbon monoxide.

The temperature in the aqueous reaction mixture may be between 10°C and 80°C, in particular between 12°C and 50°C and preferably between 15°C and 35°C.

In the following, the invention is illustrated by non-limiting examples and exemplifying embodiments.

### Examples

The general reaction setup consists of a 1L- reaction device with a carbon monoxide inlet. The reactor is also connected to a pump that allows to circulate the whole reaction mixture through an electrochemical reactor, and back again into the reaction device.

### Experiment 1:

In a 1L-reaction device, *tert*-Butanol (50 g, 0.67 mol, 1.0 equiv.) and the electrolyte 1-Butyl-3-methylimidazolium triflate (7.0 g, 0.024 mol, 3.6 Mol-%) were added to water (500 mL) at room temperature. Carbon monoxide (1 bubble/s) was bubbled through the reaction mixture, while circulating the reaction mixture through the electrochemical reactor (cathode: steel, anode: diamond, electrode distance: 1.5 cm, no separator). The electrodes were operated at 125 A/m² by applying a potential of 20 V. During the reaction, a voltage drop was observed due to HMB formation, which increases the electrical conductivity. After 2 h at room temperature, HMB was obtained in a yield of 73% (10 wt-% in reaction mixture, 58 g, 0.49 mol) as verified by HPLC analysis.

### Experiment 2:

In a 1L-reaction device, *tert-*Butanol (50 g, 0.67 mol, 1.0 equiv.) was added to water (450 mL) at room temperature. No additional electrolyte was used. Carbon monoxide (1 bubble/s) was bubbled through the reaction mixture, while circulating the reaction mixture through the electrochemical reactor (cathode: steel, anode: diamond, electrode distance: 1.5 cm, no separator). No electrical current was observed when applying a potential of 20 V. No HMB formation was observed after 2 h at room temperature.

### Experiment 3:

In a 1L-reaction device, *tert*-Butanol (50 g, 0.67 mol, 1.0 equiv.) and HMB (10 g, 0.085 mol, 13 Mol-%) as electrolyte were added to water (440 mL) at room temperature. Carbon monoxide (1 bubble/s) was bubbled through the reaction mixture, while circulating the reaction mixture through the electrochemical reactor (cathode: steel, anode: diamond, electrode distance: 1.5 cm, no separator).

The electrodes were operated at 3000 A/m² by applying a potential of 20 V. After 2 h at room temperature, an additional amount of HMB was formed as verified by HPLC analysis.

## Claims

1. A method for preparing 3-hydroxy-3-methylbutyric acid (HMB) or salts thereof from *tert*-butanol and carbon monoxide, wherein *tert*-butanol is reacted in an aqueous mixture with carbon monoxide under electrolytic conditions.

2. The method according to claim 1, wherein the electric potential to be applied to the aqueous reaction mixture is between 1.5 V and 25 V.

3. The method according to claim 1 or according to claim 2, wherein the electrodes are operated at a current density starting from 1 A/m² up to 10.000 A/m².

4. The method according to any one of the preceding claims, wherein the electric potential is applied to the aqueous reaction mixture using a diamond anode and/or a steel cathode.

5. The method according to any one of the preceding claims, the method being conducted in the presence of at least one conducting electrolyte.

6. The method according to claim 5, wherein the initial amount of the at least one conducting electrolyte present in the aqueous reaction mixture is between 1 wt.% and 20 wt.%, based on the amount of tert-butanol present in the aqueous mixture.

7. The method according to claim 5 or according to claim 6, wherein the at least one conducting electrolyte is selected from the group consisting of tetraalkyl ammonium hexaflurophosphates, tetraalkyl ammonium tetrafluoroborates, and tetraalkyl imidazolium triflates, and 3-hydroxy-3-methylbutyric acid (HMB)or salts thereof, or any combination of these conducting electrolytes.

8. The method according to any one of claims 5 to 7, wherein the at least one conducting electrolyte is selected from the group consisting of tetrabutyl ammoniumhexafluorophosphate, tetrabutyl ammoniumtetrafluoroborate 1-butyl-3-methylimidazolium triflate, and 3-hydroxy-3-methylbutyric acid (HMB) or salts thereof, or any combination of these conducting electrolytes.

9. The method according to any one of claims 5 to 8, wherein the at least one conducting electrolyte is 1-butyl-3-methylimidazolium triflate.

10. The method according to any one of the preceding claims, wherein the carbon monoxide is provided to the aqueous reaction mixture in gaseous form.

11. The method according to any one of claims 1 to 9, wherein the carbon monoxide is obtained in *situ* from gaseous carbon dioxide.

12. The method according to any one of the preceding claims, wherein the temperature in the aqueous reaction mixture is between 10°C and 80 °C

## Patentansprüche

1. Verfahren zur Herstellung von 3-Hydroxy-3-methylbuttersäure (HMB) oder Salzen davon aus tert.-Butanol und Kohlenmonoxid, wobei tert.-Butanol in einer wässrigen Mischung unter elektrolytischen Bedingungen mit Kohlenmonoxid umgesetzt wird.

2. Verfahren nach Anspruch 1, wobei die an die wässrige Reaktionsmischung anzulegende elektrische Spannung zwischen 1,5 V und 25 V liegt.

3. Verfahren nach Anspruch 1 oder nach Anspruch 2, wobei die Elektroden bei einer Stromdichte von 1 A/m² bis 10.000 A/m² betrieben werden.

4. Verfahren nach einem der vorhergehenden Ansprüche, wobei die elektrische Spannung unter Verwendung einer Diamantanode und/oder einer Stahlkathode an die wässrige Reaktionsmischung angelegt wird.

5. Verfahren nach einem der vorhergehenden Ansprüche, wobei das Verfahren in Gegenwart von mindestens einem Leitelektrolyt durchgeführt wird.

6. Verfahren nach Anspruch 5, wobei die Anfangsmenge des mindestens einen Leitelektrolyts, die in der wässrigen Reaktionsmischung vorliegt, zwischen 1 Gew.-% und 20 Gew.-%, bezogen auf die in der wässrigen Mischung vorliegende Menge von *tert*.-Butanol, liegt.

7. Verfahren nach Anspruch 5 oder nach Anspruch 6, wobei der mindestens eine Leitelektrolyt aus der Gruppe bestehend aus Tetraalkylammoniumhexafluorophosphaten, Tetraalkylammoniumtetrafluoroboraten und Tetraalkylimidazoliumtriflaten und 3-Hydroxy-3-methylbuttersäure (HMB) oder Salzen davon oder einer beliebigen Kombination dieser Leitelektrolyte ausgewählt wird.

8. Verfahren nach einem der Ansprüche 5 bis 7, wobei der mindestens eine Leitelektrolyt aus der Gruppe bestehend aus Tetrabutylammoniumhexafluorophosphat, Tetrabutylammoniumtetrafluoroborat, 1-Butyl-3-methylimidazoliumtriflat und 3-Hydroxy-3-methylbuttersäure (HMB) oder Salzen davon oder einer beliebigen Kombination dieser Leitelektrolyte ausgewählt wird.

9. Verfahren nach einem der Ansprüche 5 bis 8, wobei es sich bei dem mindestens einen Leitelektrolyt um 1-Butyl-3-methylimidazoliumtriflat handelt.

10. Verfahren nach einem der vorhergehenden Ansprüche, wobei das Kohlenmonoxid der wässrigen Reaktionsmischung in gasförmiger Form zugeführt wird.

11. Verfahren nach einem der Ansprüche 1 bis 9, wobei das Kohlenmonoxid in situ aus gasförmigem Kohlendioxid erhalten wird.

12. Verfahren nach einem der vorhergehenden Ansprüche, wobei die Temperatur in der wässrigen Reaktionsmischung zwischen 10 °C und 80 °C liegt.

## Revendications

1. Procédé de préparation d'acide 3-hydroxy-3-méthylbutyrique (HMB) ou de sels correspondants à partir de tert-butanol et de monoxyde de carbone, le tert-butanol étant mis à réagir dans un mélange aqueux avec du monoxyde de carbone dans des conditions électrolytiques.

2. Procédé selon la revendication 1, le potentiel électrique à appliquer au mélange réactionnel aqueux étant compris entre 1,5 V et 25 V.

3. Procédé selon la revendication 1 ou selon la revendication 2, les électrodes étant utilisées à une densité de courant démarrant à 1 A/m² jusqu'à 10,000 A/m².

4. Procédé selon l'une quelconque des revendications précédentes, le potentiel électrique étant appliqué au mélange réactionnel aqueux à l'aide d'une anode en diamant et/ou d'une cathode en acier.

5. Procédé selon l'une quelconque des revendications précédentes, le procédé étant conduit en présence d'au moins un électrolyte conducteur.

6. Procédé selon la revendication 5, la quantité initiale de l'au moins un électrolyte conducteur présent dans le mélange réactionnel aqueux étant comprise entre 1 % en poids et 20 % en poids, sur la base de la quantité de tert-butanol présente dans le mélange aqueux.

7. Procédé selon la revendication 5 ou selon la revendication 6, l'au moins un électrolyte conducteur étant choisi dans le groupe constitué par des hexaflurophosphates de tétraalkylammonium, des tétrafluoroborates de tétraalkylammonium et des triflates de tétraalkylimidazolium, et l'acide 3-hydroxy-3-méthylbutyrique (HMB) ou des sels correspondants, ou une quelconque combinaison de ces électrolytes conducteurs.

8. Procédé selon l'une quelconque des revendications 5 à 7, l'au moins un électrolyte conducteur étant choisi dans le groupe constitué par l'hexafluorophosphate de tétrabutylammonium, le tétrafluoroborate de tétrabutylammonium, le triflate de 1-butyl-3-méthylimidazolium et l'acide 3-hydroxy-3-méthylbutyrique (HMB) ou des sels correspondants, ou une quelconque combinaison de ces électrolytes conducteurs.

9. Procédé selon l'une quelconque des revendications 5 à 8, l'au moins un électrolyte conducteur étant le triflate de 1-butyl-3-méthylimidazolium.

10. Procédé selon l'une quelconque des revendications précédentes, le monoxyde de carbone étant fourni au mélange réactionnel aqueux sous forme gazeuse.

11. Procédé selon l'une quelconque des revendications 1 à 9, le monoxyde de carbone étant obtenu in *situ* à partir de dioxyde de carbone gazeux.

12. Procédé selon l'une quelconque des revendications précédentes, la température dans le mélange réactionnel aqueux étant comprise entre 10 °C et 80 °C.
